Europäisches Patentamt

European Patent Office

Office européen des brevets

(11) Veröffentlichungsnummer: **0 256 462 B1**

(19)

## (12) EUROPÄISCHE PATENTSCHRIFT

(45) Veröffentlichungstag der Patentschrift: **21.10.92**

(21) Anmeldenummer: **87111512.7**

(22) Anmeldetag: **08.08.87**

Die Akte enthält technische Angaben, die nach dem Eingang der Anmeldung eingereicht wurden und die nicht in dieser Patentschrift enthalten sind.

(51) Int. Cl.⁵: **A61K 7/09**

(54) **Verfahren zur dauerhaften Haarverformung.**

(30) Priorität: **16.08.86 DE 3627746**

(43) Veröffentlichungstag der Anmeldung:
**24.02.88 Patentblatt 88/08**

(45) Bekanntmachung des Hinweises auf die Patenterteilung:
**21.10.92 Patentblatt 92/43**

(84) Benannte Vertragsstaaten:
**AT BE CH DE ES FR GB IT LI NL SE**

(56) Entgegenhaltungen:
**EP-A- 0 114 414**
**EP-A- 0 134 452**
**BE-A- 680 599**

**PATENT ABSTRACTS OF JAPAN, Band 9, Nr. 241 (C-306)[1964], 27 September 1985**

**CHEMICAL ABSTRACTS, Band 104, Nr. 2, Januar 1986, Columbus, OH (US); Seite 300, Nr. 10376g**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Klenk, Adolf, Dr.**
**An den Pappeln 14**
**W-4048 Grevenbroich 5(DE)**
Erfinder: **Hollenberg, Detlef, Dr.**
**Hugo Wolff-Str. 15**
**W-4010 Hilden(DE)**
Erfinder: **Höffkes, Horst, Dr.**
**Carlo Schmid-Str. 113**
**W-4000 Düsseldorf(DE)**

**Beschreibung**

Die Erfindung betrifft ein Verfahren zur dauerhaften Haarverformung durch reduktive Spaltung und erneute oxidative Knüpfung von Disulfidbindungen des Haarkeratins, bei welchem durch eine Nachbehandlung mit einer Zubereitung, die ein Aluminium(III)-Salz und eine Hydroxydi- oder tricarbonsäure mit 4 bis 6 C-Atomen enthält, eine Verstärkung der Wellwirkung erzielt wird.

Die dauerhafte Haarverformung wird nach den bekannten Dauerwell-Verfahren in der Weise durchgeführt, daß man das Haar mechanisch verformt und die Verformung z. B. durch Aufwickeln auf Haarwickler oder Papilloten festlegt. Vor und/oder nach dieser Verformung behandelt man das Haar mit der wäßrigen Zubereitung einer keratinreduzierenden Substanz, spült nach einer Einwirkungszeit mit Wasser und behandelt dann in einem zweiten Schritt mit der wäßrigen Zubereitung eines Oxidationsmittels. Nach einer Einwirkungszeit wird auch dieses aus dem Haar ausgespült und das Haar von den mechanischen Verformungshilfsmitteln (Wickler, Papilloten) befreit.

Die wäßrige Zubereitung des Keratinreduktionsmittels ist üblicherweise stark alkalisch eingestellt, damit das Haar quillt und auf diese Weise ein tiefes Eindringen der keratinreduzierenden Substanz in das Haar ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung des Haars durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Weder die quellende Wirkung des Alkali noch die keratinspaltende Wirkung des Reduktionsmittels lassen sich gänzlich rückgängig machen, das frisch dauergewellte Haar ist daher sehr empfindlich gegen mechanische Beanspruchung. Eine zu starke Reduktionsstufe - z. B. infolge zu hoher Konzentration an keratinreduzierender Substanz oder zu langer Einwirkungszeit - kann auch zu starker Haarschädigung mit der Folge einer kosmetisch unerwünschten Überkräuselung des Haars führen.

Es bestand daher die Aufgabe, ein Verfahren der dauerhaften Haarverformung zu finden, bei welchem die Gefahr unerwünschter Nebenwirkungen mit größerer Sicherheit vermieden wird und bei welchem das Haar weniger stark strapaziert wird. Eine solche Möglichkeit wäre gegeben, wenn es gelänge, die Konzentration der keratinreduzierenden Substanz in der Reduktionsstufe zu verringern und durch weniger haarschädigende Maßnahmen die Haarverformung zu verstärken.

Aus JP-A-60 100 512 ist bekannt, im Rahmen von Dauerwellbehandlungen die Wirkung von Cystein in Reaktionsmitteln durch Zugabe anorganischer oder organischer Aluniniumverbindungen zu steigern. Als Lösungsvermittler für diese Aluminiumverbindungen können beispielsweise Citronensäure und Apfelsäure verwendet werden.

In US-PS 3 266 994 und EP-A-134 452 wurde vorgeschlagen, der oxidativen Fixierlösung ein wasserlösliches Magnesium-, Aluminium- oder Calciumsalz zuzusetzen. Aus US-PS 3 981 312 war ein Verfahren zur dauerhaften Haarverformung bekannt, bei welchem zwischen der Reduktionsstufe und der oxidativen Fixierung eine Behandlung des Haars mit einer Festigerlösung vorgesehen ist, die ein mehrwertiges Metallsalz in wäßriger Lösung enthält.

Aus JP-A-60 158 105 sind Haarspülungen bekannt, die organische Säuren, beispielsweise Citronensäure, enthalten.

Aus US-PS 4 409 204 war schließlich ein Nachbehandlungsmittel für dauergewelltes Haar bekannt, welches Glyoxylsäure und bestimmte ungesättigte Carbonsäuren enthält. Durch diese Maßnahmen sollte bereits dem Festigkeitsverlust des Haars durch die Dauerwellbehandlung entgegengewirkt werden. Durch die genannten Maßnahmen wurde jedoch keine befriedigende Verbesserung erreicht.

Es wurde nun gefunden, daß eine wesentliche Verbesserung der Struktur und Festigkeit dauergewellter Haare durch eine Nachbehandlung unmittelbar im Anschluß an die oxidative Fixierung erreicht wird, wenn dafür eine wäßrige Zubereitung mit einem Gehalt an gelösten Aluminium(III)-Salz und an einer Hydroxydi- oder tricarbonsäure verwendet wird.

Gegenstand der Erfindung ist daher ein Verfahren zur dauerhaften Haarverformung, bei welchem man das Haar vor und/oder nach einer mechanischen Verformung mit der wäßrigen Zubereitung einer keratinreduzierenden Substanz behandelt, das so behandelte Haar nach einer Einwirkungszeit mit Wasser spült und durch Behandlung mit einer wäßrigen Zubereitung eines Oxidationsmittels fixiert, und das so behandelte Haar nach einer Einwirkungszeit mit Wasser spült, dadurch gekennzeichnet, daß man im Anschluß an die oxidative Fixierung das noch mechanisch verformte Haar mit einer wäßrigen Zubereitung nachbehandelt, die ein gelöstes Aluminium(III)-Salz und eine Hydroxydi- oder tricarbonsäure mit 4 bis 6 C-Atomen enthält und dann die mechanischen Verformungshilfsmittel (Haarwickler oder Papilloten) entfernt.

Durch das erfindungsgemäße Verfahren wird das Ergebnis der Haarverformung so sehr verbessert, daß

2

weniger konzentrierte Zubereitungen des Keratinreduktionsmittels und/oder kürzere Einwirkungszeiten dieser Reduktionsmittel bereits zu sehr befriedigenden Haarverformungsergebnissen führen. Auf diese Weise läßt sich eine verstärkte Haarverformung unter weitgehender Schonung des Haars und unter Erhalt der natürlichen Festigkeit des Haares erzielen.

Als keratinreduzierende Substanzen können in das erfindungsgemäße Verfahren die dafür üblichen Merkaptane und die Alkalisalze der schwefligen Säure eingesetzt werden. Bevorzugt geeignet für das erfindungsgemäße Verfahren sind die Alkali- oder Ammoniumsalze der Thioglycolsäure und/oder der Thiomilchsäure. Diese können in die keratinreduzierenden Zubereitungen der ersten Stufe des erfindungsgemäßen Haarverformungsverfahrens in einer Konzentration von 0,5 bis 1,0 Mol/kg bei einem pH-Wert von 6,5 bis 10 eingesetzt werden. Die Einwirkungszeit für die keratinreduzierende Zubereitung beträgt in der Regel 20 bis 40 Minuten, wobei die Dicke des zu behandelnden Haars, der gewünschte Verformungsgrad, die Größe der verwendeten mechanischen Verformungshilfe (Haarwickler) und die Art des Keratinreduktionsmittels weitere Einflußgrößen sind. Diese Einwirkungszeit kann bei sonst gleichen Arbeitsbedingungen um 10 bis 50 % verkürzt werden, wenn die erfindungsgemäße Nachbehandlung durchgeführt wird.

Außer der keratinreduzierenden Substanz können in der wäßrigen Zubereitung zur Durchführung der ersten Stufe des erfindungsgemäßen Haarverformungsverfahrens alle hierfür bekannten Hilfsmittel und Zusatzstoffe enthalten sein. Dies sind in erster Linie oberflächenaktive Stoffe, Komplexbildner, Ammoniak und Puffersalze zur Einstellung eines pH-Wertes von 6,5 bis 10, z. B. Ammoniumcarbonat, Harnstoff, Glycerin, wasserlösliche Polymere zur Viskositätserhöhung, Duftstoffe, Farbstoffe und Trübungsmittel.

In der wäßrigen Zubereitung zur Durchführung der zweiten Stufe, der oxidativen Fixierung, können als Oxidationsmittel z. B. Wasserstoffperoxid und die zur Stabilisierung wäßriger Wasserstoffperoxidzubereitungen üblichen Stabilisatoren enthalten sein. Der pH-Wert solcher wäßriger $H_2O_2$-Zubereitungen, die etwa 0,5 bis 3,0 Gew.-% $H_2O_2$ enthalten, liegt bevorzugt bei 2 bis 4, er wird durch anorganische Säuren, bevorzugt Phosphorsäure eingestellt. Das bevorzugte Oxidationsmittel ist Natrium- oder Kaliumbromat. Solche Bromate werden in Konzentrationen von 1 bis 10 Gew.-% eingesetzt und der pH-Wert der Lösungen wird auf 4 bis 7 eingestellt.

Außer dem Oxidationsmittel können in der wäßrigen Zubereitung zur Durchführung der zweiten Stufe des erfindungsgemäßen Haarverformungsverfahrensweitere hierfür bekannte Hilfsmittel und Zusätze enthalten sein: Dies sind z. B. oberflächenaktive Stoffe, quartäre Ammoniumsalze, kationische Polymere, wasserlösliche Proteine oder Proteinderivate, Duftstoffe und Trübungsmittel.

Die wäßrigen Zubereitungen zur Durchführung der ersten und/oder zweiten Stufe des erfindungsgemäßen Dauerwellverfahrens und/oder der Nachbehandlung können auch in Form von Schaumaerosolen konfektioniert sein, die mit einem verflüssigten Gas wie z. B. Propan-Butan-Gemischen, $N_2O$, Dimethylether, Fluorkohlenwasserstoffreibmitteln oder Gemischen davon in Aerosolbehältern mit Schaumventil abgefüllt werden.

Die wäßrige Zubereitung zur Durchführung der Nachbehandlung enthält als obligatorische Komponenten ein gelöstes Aluminium(III)-Salz und eine Hydroxydi- oder tricarbonsäure mit 4 bis 6 C-Atomen. Als Aluminium(III)-Salze können z. B. $AlCl_3$, $AlCl_3 \cdot 6\ H_2O$, $Al_2(SO_4)_3$, $Al_2(SO_4)_3 \cdot 18\ H_2O$, $AlNa(SO_4)_2 \cdot 12\ H_2O$, $Al(NH_4)(SO_4)_2 \cdot 12\ H_2O$ und andere wasserlösliche Aluminiumsalze, z. B. Aluminiumlactat, Aluminiumacetat oder Aluminiumsalze anderer organischer Carbonsäuren verwendet werden. Das Aluminium(III)-Salz wird in einer Menge von 10 bis 1 000 mMol/kg berechnet als Al eingesetzt. Bevorzugt geeignet ist $AlCl_3$, welches bevorzugt in einer Menge von 0,2 bis 5 Gew.-% in die wäßrige Zubereitung eingesetzt wird. Als Hydroxydi- oder tricarbonsäure eignen sich Äpfelsäure, Weinsäure und Citronensäure. Von den Hydroxydi- und tricarbonsäuren mit Asymmetriezentren eignen sich jeweils beide optischen Antipoden sowie die Razemate und im Falle der Weinsäure auch die Meso-Form. Die Hydroxydi- oder tricarbonsäuren werden in einer Menge von 0,5 bis 5 Gew.-% der wäßrigen Zubereitung zugesetzt. Der pH-Wert der Zubereitung sollte zwischen 2 und 6 liegen.

Bevorzugt geeignet ist Weinsäure in ihrer D- und L-Form und in der Form des Razemats, der Traubensäure.

Die wäßrige Zubereitung zur Durchführung der 3. Stufe des erfindungsgemäßen Haarverformungsverfahrens kann als Haarfestiger, als Konditioniermittel, als Haarwasser oder als emulsionsförmige Spülung konfektioniert sein. Sie kann neben den genannten kennzeichnenden Komponenten alle für solche Zubereitungen üblichen Hilfsmittel und Zusätze enthalten.

Für Haarfestiger sind dies wasserlösliche Polymere mit haarfestigender Wirkung wie z. B. Polyvinylpyrrolidon und Polyvinylpyrrolidon-vinylacetat-Copolymere, Methyl- und Hydroxyethylcellulose, Carboxymethylcellulose, Polyacrylsäuren und wasserlösliche Pflanzengumme. Schließlich können auch niedere Alkohole, Duftstoffe und Parfümölsolubilisatoren enthalten sein.

Für Konditioniermittel kommen als weitere Komponenten z. B. haarfestigende Zusätze, Glucose, Vitamine, Proteinderivate, Pflanzenextrakte, Duftstoffe und Parfümölsolubilisatoren in Frage.

Für Haarwässer können als weitere Komponenten niedere Alkohole, Pflanzenextrakte, kosmetische Ölkomponenten, kationische Tenside, Duftstoffe und Parfümölsolubilisatoren verwendet werden.

In Spülungen mit pfrlegender und konditionierender Wirkung können Fettalkohole wie Cetyl- und Stearylalkohol, Fettsäuremono-und Diglyceride z. B. auf Basis von Palmitin- und Stearinsäure, kosmetische Ölkomponenten, Emulgatoren, kationische Tenside wie z. B. Cetyltrimethylammoniumchlorid oder Cetylpyridiniumchlorid sowie Duftstoffe als weitere Komponenten enthalten sein.

Bevorzugt wird die Zubereitung zur Nachbehandlung in Form eines Konditioniermittels konfektioniert, das neben dem Aluminium(III)-Salz und der Hydroxydi- oder tricarbonsäure eine quarternäre Ammoniumverbindung mit avivierender Wirkung in einer Menge von 0,5 bis 3 Gew.-% enthält.

Da die wäßrigen Zubereitungen zur Durchführung des erfindungsgemäßen Verfahrens unmittelbar nacheinander auf das mechanisch verformte und durch Wickler oder Papilloten fixierte Haar angewendet werden und der Erfolg der Nachbehandlung wesentlich davon abhängig ist, daß diese unmittelbar im Anschluß an die oxidative Fixierung an dem noch mechanisch verformten, gewickelten Haar durchgeführt wird, ist es besonders vorteilhaft, wenn die Zubereitungen zur Durchführung des erfindungsgemäßen Verfahrens in einer Verpackungseinheit konfektioniert sind. Auf diese Weise läßt sich vor allem auch für die nicht professionelle Heimanwendung des Verfahrens eine größere Sicherheit dafür schaffen, daß der Anwender in Menge und Konzentration optimal aufeinander abgestimmte Zubereitungen verwendet und Fehldosierungen weitgehend vermieden werden. Ein weiterer Erfindungsgegenstand sind daher Mittel zur Durchführung des Verfahrens zur dauerhaften Haarverformung, bestehend aus drei räumlich getrennten Zubereitungen, die in einer Verpackungseinheit konfektioniert sind, wobei die erste Zubereitung eine keratinreduzierende Substanz, die zweite Zubereitung ein Oxidationsmittel und die dritte Zubereitung ein Aluminium(III)-Salz und eine Hydroxydi- oder tricarbonsäure mit 4 bis 6 C-Atomen enthält.

Die folgenden Beispiele sollen den Gegenstand der Erfindung näher erläutern:

**Beispiele**

| 1. Keratinreduzierende Zubereitung | |
| --- | --- |
| Thioglycolsäure | 7,5 Gew.-% |
| Ammoniak (konz. wäßrige Lösung) | 9,0 Gew.-% |
| 1-Hydroxyethan-1,1-diphosphonsäure | 0,3 Gew.-% |
| Ricinusöloxethylat (40 EO) | 3,0 Gew.-% |
| Nonylphenoloxethylat (9 EO) | 3,0 Gew.-% |
| Ammoniumcarbonat | 1,0 Gew.-% |
| Opacifier Permanent 601 (1) | 0,3 Gew.-% |
| Parfümöl | 0,5 Gew.-% |
| Wasser | ad 100,0 Gew.-% |

| 2. Oxidative Fixierung | |
| --- | --- |
| Kaliumbromat | 3,5 Gew.-% |
| Weinsäure | 0,15 Gew.-% |
| Kokosfettsäurediethanolamid | 5,0 Gew.-% |
| Texapon N 25 (2) | 25,0 Gew.-% |
| Parfümöl | 0,3 Gew.-% |
| Natriumchlorid | 1,0 Gew.-% |
| Wasser | ad 100,0 Gew.-% |

Zubereitungen zur Nachbehandlung nach der oxidativen Fixierung einer Dauerwellbehandlung:

| 3.1 Dauerwell-Haarfestiger | |
|---|---|
| Luviskol VA 64 (3) | 1,5 Gew.-% |
| Weinsäure | 4,0 Gew.-% |
| AlCl$_3$ | 2,0 Gew.-% |
| Isopropanol | 40,0 Gew.-% |
| Parfümöl | 0,5 Gew.-% |
| Wasser | ad 100,0 Gew.-% |

| 3.2 Dauerwell-Konditioniermittel | |
|---|---|
| Ricinusöloxethylat (40 EO) | 0,2 Gew.-% |
| D-Panthenol | 3,0 Gew.-% |
| Glucose | 3,0 Gew.-% |
| Zitronensäure | 3,0 Gew.-% |
| AlCl$_3$ | 3,0 Gew.-% |
| Opacifier E 305 (1) | 0,1 Gew.-% |
| Parfümöl | 0,1 Gew.-% |
| Wasser | ad 100,0 Gew.-% |

| 3.3 Dauerwell-Haarwasser | |
|---|---|
| Cetiol HE (4) | 1,0 Gew.-% |
| Extrapon Birke spezial (5) | 1,0 Gew.-% |
| Äpfelsäure | 3,0 Gew.-% |
| Al$_2$(SO$_4$)$_3$ | 2,0 Gew.-% |
| Isopropanol | 30,0 Gew.-% |
| Parfümöl | 0,3 Gew.-% |
| Wasser | ad 100,0 Gew.-% |

| 3.4 Dauerwell-Spülung | |
|---|---|
| Cetyl/Stearylalkohol | 4,0 Gew.-% |
| Cetyltrimethylammoniumchlorid (25%ige wäßrige Lösung) | 1,5 Gew.-% |
| AlCl$_3$ | 3,0 Gew.-% |
| Weinsäure | 4,0 Gew.-% |
| Copaiva-Balsamöl | 0,4 Gew.-% |
| Parfüm | 0,4 Gew.-% |
| Natriumlaurylsulfat | 1,0 Gew.-% |
| Wasser | ad 100,0 Gew.-% |

In den Rezepturen wurden die folgenden Handelsprodukte verwendet:

(1) Styrol-Copolymer-Dispersion, Trübungsmittel (Morton Williams)

(2) Fettalkohol-C$_{12}$-C$_{14}$-polyglycolether(2EO)-sulfat, N-Salz, 28%ige wäßrige Lösung (Henkel KGaA)

(3) Vinylpyrrolidon-Vinylacetat-(60:40)-Copolymer (BASF)

(4) Fettsäureester eines Glycerinoxethylats, CTFA-Bezeichnung: PEG-7-Glyceryl Cocoate (Henkel KGaA)

(5) Birken-Wirkstoff-Konzentrat (Dragoco)

**Patentansprüche**

1.  Verfahren zur dauerhaften Haarverformung, bei welchem man das Haar vor und/oder nach einer mechanischen Verformung mit der wäßrigen Zubereitung einer keratinreduzierenden Substanz behan-

5

delt, das so behandelte Haar nach einer Einwirkungszeit mit Wasser spült und durch Behandlung mit einer wäßrigen Zubereitung eines Oxidationsmittels fixiert, und das so behandelte Haar nach einer Einwirkungszeit mit Wasser spült, dadurch gekennzeichnet, daß man im Anschluß an die oxidative Fixierung das noch mechanisch verformte Haar mit einer wäßrigen Zubereitung nachbehandelt, die ein gelöstes Aluminium(III)-Salz und eine Hydroxydi- oder tricarbonsäure mit 4 bis 6 C-Atomen enthält.

2. Verfahren nach Anspruch 1, dadurch gekennzeichnet, daß als keratinreduzierende Substanz ein Alkali- oder Ammonium-Thioglycolat oder Thiolactat verwendet wird.

3. Verfahren nach den Ansprüchen 1 oder 2, dadurch gekennzeichnet, daß als Oxidationsmittel Kalium- oder Natriumbromat oder Wasserstoffperoxid verwendet wird.

4. Verfahren nach den Ansprüchen 1 bis 3, dadurch gekennzeichnet, daß die Zubereitung zur Nachbehandlung 10 bis 1 000 mMol/kg, berechnet als Al eines Aluminium(III)-Salzes und 0,5 bis 5 Gew.-% Weinsäure enthält und einen pH-Wert zwischen 2 und 6 aufweist.

5. Mittel zur Durchführung des Verfahrens nach den Ansprüchen 1 bis 4, bestehend aus drei räumlich getrennten Zubereitungen, die in einer Verpackungseinheit konfektioniert sind, wobei die erste Zubereitung eine keratinreduzierende Substanz, die zweite Zubereitung ein Oxidationsmittel und die dritte Zubereitung ein Aluminium(III)-Salz und eine Hydroxydi- oder tricarbonsäure mit 4 bis 6 C-Atomen enthält.

**Claims**

1. A process for the permanent shaping of hair, in which the hair is treated with an aqueous preparation of a keratin-reducing substance before and/or after a mechanical shaping step, the hair thus treated is rinsed with water after a certain contact time and fixed by treatment with an aqueous preparation of an oxidizing agent and the hair thus treated is rinsed with water after a certain contact time, characterized in that, after the oxidative fixing step, the mechanically shaped hair is aftertreated with an aqueous preparation containing a dissolved aluminium(III) salt and a $C_{4-6}$ hydroxydi- or tricarboxylic acid.

2. A process as claimed in claim 1, characterized in that an alkali metal or ammonium thioglycolate or thiolactate is used as the keratin-reducing substance.

3. A process as claimed in claim 1 or 2, characterized in that potassium or sodium bromate or hydrogen peroxide is used as the oxidizing agent.

4. A process as claimed in claims 1 to 3, characterized in that the aftertreatment preparation contains from 10 to 1000 mmol/kg, expressed as Al, of an aluminium(III) salt and from 0.5 to 5% by weight of tartaric acid and has a pH value of from 2 to 6.

5. Means for carrying out the process claimed in claims 1 to 4 consisting of three spatially separate preparations accommodated in a single pack, the first preparation containing a keratin-reducing substance, the second preparation an oxidizing agent and the third preparation an aluminium(III) salt and a $C_{4-6}$ hydroxydi- or tricarboxylic acid.

**Revendications**

1. Procédé de déformation permanente des cheveux dans lequel on traite les cheveux avant et/ou après une déformation mécanique avec la préparation aqueuse d'une substance qui réduit la kératine, on rince les cheveux ainsi traités après une durée d'action avec de l'eau, et on fixe par traitement avec une préparation aqueuse d'un agent d'oxydation et on rince les cheveux ainsi traités après une durée d'action avec de l'eau, procédé caractérisé en ce que l'on traite ultérieurement en connexion avec la fixation oxydative les cheveux encore déformés mécaniquement avec une préparation aqueuse renfermant un sel d'aluminium (III) dissout et un acide hydroxydicarboxylique ou tricarboxylique ayant de 4 à 6 atomes de carbone.

2. Procédé selon la revendication 1, caractérisé en ce que comme substance qui réduit la kératine, on utilise un thioglycolate ou un thiolactate alcalin ou d'ammonium.

3. Procédé selon les revendications 1 ou 2, caractérisé en ce que comme agent d'oxydation on utilise le bromate de potassium ou de sodium, ou le peroxyde d'hydrogène.

4. Procédé selon les revendications 1 à 3, caractérisé en ce que la préparation pour le traitement ultérieur renferme de 10 à 1 000 mmol/kg, calculées en Al, d'un sel d'aluminium et de 0,5 à 5% en poids d'acide tartrique et possède une valeur de pH comprise entre 2 et 6.

5. Moyen pour l'exécution du procédé selon les revendications 1 à 4 qui se compose de trois préparations séparées dans l'espace qui sont conditionnées dans une unité d'emballage, dans laquelle la première préparation contient une substance réductrice de la kératine, la deuxième préparation contient un agent d'oxydation et la troisième préparation renferme un sel d'aluminium (III) et un acide hydroxydicarboxylique ou tricarboxylique ayant de 4 à 6 atomes de carbone.